# EUROPEAN PATENT SPECIFICATION

(11) **EP 1 275 651 B1**
(45) Date of publication and mention of the grant of the patent: **31.08.2005**
(21) Application number: 02013425.0
(22) Date of filing: 13.06.2002
(51) Int. Cl.: C07D 495/14, C07D 519/00, C09K 19/34, H01B 1/12

(54) **Thienothiophene derivatives**
Thienothiophen-Derivate
Dérivés de thiénothiophènes

(30) Priority: 09.07.2001 EP 01115742
(43) Date of publication of application: 15.01.2003
(73) Proprietor: Merck Patent GmbH, 64293 Darmstadt (DE)
(72) Inventor: Farrand, Louise, Blandford Forum, Dorset DT11 9ED (GB); Thompson, Marcus, Fordingbridge, Hampshire SP6 1RR (GB); Giles, Mark, Southampton SO15 2LE (GB); Heeney, Martin, Southampton SO14 6TQ (GB); Tierney, Steven, Southampton SO15 7QW (GB); Shkunov, Maxim, Southampton SO16 6SX (GB); Sparrowe, David, Bournemouth, Dorset BH6 5EJ (GB); McCulloch, Iain, Kings Somborne, Hampshire SO20 6PE (GB)

(56) References cited:
- WO-A-99/12989
- KOSSMEHL G ET AL: "Über Polyarylenalkenylene und Polyheteroarylenalkenylene" MAKROMOLEKULARE CHEMIE, MACROMOLECULAR CHEMISTRY AND PHYSICS, HUTHIG UND WEPF VERLAG, BASEL, CH, vol. 183, no. 11, 1 November 1982 (1982-11-01), pages 2771-2786, XP002085568 ISSN: 0025-116X
- HARISTOY D ET AL: "STRUCTURE AND PHOTOCONDUCTIVE BEHAVIOUR OF A SANIDIC LIQUID CRYSTAL" LIQUID CRYSTALS, TAYLOR AND FRANCIS LTD, LONDON, GB, vol. 27, no. 3, March 2000 (2000-03), pages 321-328, XP000932241 ISSN: 0267-8292
- KIEBOOMS R ET AL: "SYNTHESIS, ELECTRICAL, AND OPTICAL PROPERTIES OF CONJUGATED POLYMERS" HANDBOOK OF ADVANCED ELECTRONIC AND PHOTONIC MATERIALS AND DEVICES, XX, XX, vol. 8, 2001, pages 1-102, XP001029240

## Description

### Field of Invention

The invention relates to new thienothiophene derviatives. The invention further relates to their use as semiconductors or charge transport materials, in optical, electrooptical or electronic devices like for example organic field effect transistors (FET or OFET) for thin film transistor liquid crystal displays and integrated circuit devices such as radio frequency identification (RFID) tags, electroluminescent devices in flat panel displays, and in photovoltaic and sensor devices. The invention further relates to a field effect transistor, light emitting device or identification (ID) tag comprising the new thienothiophene derivatives.

### Background and Prior Art

Organic materials have recently shown promise as the active layer in organic based thin film transistors and organic field effect transistors [see H. E. Katz, Z. Bao and S. L. Gilat, *Acc. Chem. Res.,* 2001, **34**, 5, 359]. Such devices have potential applications in smart cards, security tags and the switching element in flat panel displays. Organic materials are envisaged to have substantial cost advantages over their silicon analogues if they can be deposited from solution, as this enables a fast, large-area fabrication route.

The performance of the device is principally based upon the charge carrier mobility of the semiconducting material and the current on/off ratio, so the ideal semiconductor should have a low conductivity in the off state, combined with a high charge carrier mobility (> 1 x 10⁻³ cm²V⁻¹ s⁻¹). In addition, it is important that the semiconducting material is relatively stable to oxidation i.e. it has a high ionisation potential, as oxidation leads to reduced device performance.

Compounds known in prior art for use as semiconductors are the fused dimer of dithienothiophene (DTT) and α,α'-bis(dithieno[3,2-b:2',3'-d]thiophene (BDT) having the structures shown below.

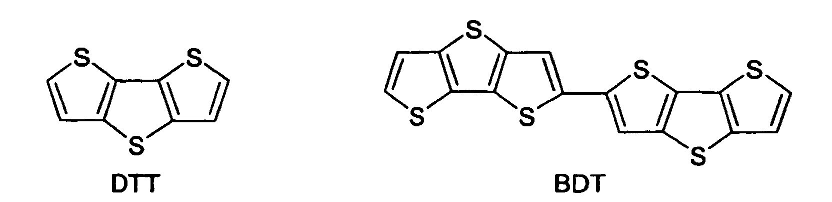

BDT and DDT are described for example in F. de Jong and M. J. Janssen, J. Org. Chem., 1971, 36, 12, 1645; S. Inaoka and D. M. Collard, J. Mater. Chem., 1999, 9, 1719; H. Sirringhaus et al, Appl. Phys. Lett. 1997, 71 (26), 3871; X-C. Li et al, J. Am. Chem. Soc., 1998, 120, 2206, and in the international patent application WO 99/12989.

In particular BDT, which has been extensively studied, has been shown to be an effective p-type semiconductor for organic FETs with a very high charge carrier mobility of 0.02-0.05 cm²/V. BDT also has been found in the solid state to have a completely coplanar formation, and to be more planar than oligomers of thiophene.

However, the materials described in prior art have several disadvantages., BDT has a high melting point and is very insoluble, therefore, if used as the active layer in an organic thin film transistor, it cannot be readily solution processed.

As a result, for applications like FETs, prior art materials like BDT are usually deposited as a thin film by vacuum deposition, which is an expensive processing technique that is unsuitable for the fabrication of large-area films.

It was an aim of the present invention to provide new organic materials for use as semiconductors or charge transport materials, which are easy to synthesize, have high charge mobility, and good processibility. The materials should be easily processible to form thin and large-area films for use in semiconductor devices. Other aims of the invention are immediately evident to those skilled in the art from the following description.

It was found that these aims can be achieved by providing thienothiophenes as described below.

US 4,639,328 discloses compounds with a thienothiophene group for use as components of liquid crystalline phases, but does not proivde any suggestion to semiconductor materials.

### Definition of Terms

The terms 'liquid crystalline or mesogenic material' or 'liquid crystalline or mesogenic compound' means materials or compounds comprising one or more rod-shaped, lath-shaped or disk-shaped mesogenic groups, i.e. groups with the ability to induce liquid crystal phase behaviour. The compounds or materials comprising mesogenic groups do not necessarily have to exhibit a liquid crystal phase themselves. It is also possible that they show liquid crystal phase behaviour only in mixtures with other compounds.

### Summary of the Invention

One object of the invention are thienothiophene derivatives of formula I

R¹-Z¹-(A¹-Z²)ₘ-(T-Z³)ₙ-(A²-Z⁴)ₒ-R² (I)

wherein
- R¹ and R²: are independently of each other straight chain, or branched alkyl with 1 to 20 C-atoms, which may be unsubstituted, mono- or poly-substituted by F, Cl, Br, I or CN, it being also possible for one or more non-adjacent CH₂ groups to be replaced, in each case independently from one another, by -O-, -S-, -NH-, -NR⁰-, -SiR⁰R⁰⁰-, -CO-, -COO-, -OCO-, -OCO-O-, -S-CO-, -CO-S-, -CH=CH- or -C≡C- in such a manner that O and/or S atoms are not linked directly to one another,
- R⁰ and R⁰⁰: are independently of each other H or alkyl with 1 to 12 C-atoms,
- A¹ and A²: are independently of each other 1,4-phenylene or thiophene-2,5-diyl, it being possible for these groups to be unsubstituted, mono- or polysubstituted by L, with L being halogen, CN, SCN, NO₂, SF₅ or an alkyl, alkoxy, alkylcarbonyl or alkoxycarbonyl group with 1 to 12 C atoms, wherein one or more H atoms may be substituted with F or Cl,
- Z¹, Z², Z³ and Z⁴: are independently of each other -CH=CR⁰-, -CX¹=CX²-, -C≡C- or a single bond,
- X¹ and X²: are independently of each other H, F, Cl or CN,
- T: is a group of formula IIa 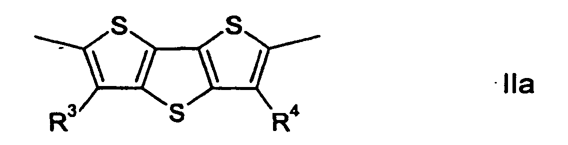
- R³ and R⁴: are H or have one of the meanings of R¹,
- m and o: are independently of each other 1, 2 or 3, and
- n: is 1.

Another object of the invention is the use of compounds of formula I as semiconductors or charge transport materials, in particular in optical, electrooptical or electronic devices, like for example components of integrated circuitry, field effect transistors (FET) for example as thin film transistors in flat panel display applications or for Radio Frequency Identification (RFID) tags, or in semiconducting components for organic light emitting diode (OLED) applications such as electroluminescent displays or backlights of flat panel displays, for photovoltaic or sensor devices, as electrode materials in batteries, as photoconductors and for electrophotographic applications.

Another object of the invention is a field effect transistor, for example as a component of integrated circuitry, as a thin fim transistor in flat panel display applications, or in an RFID tag, comprising one or more compounds of formula I.

Another object of the invention is a semiconducting component, for example in OLED applications like electroluminescent displays or backlights of flat panel displays, in photovoltaic or sensor devices, as electrode materials in batteries, as photoconductors and for electrophotographic applications, comprising one or more compounds of formula I.

Another object of the invention is a security marking or device comprising an RFID or ID tag or a FET according to the invention.

### Detailed Description of the Invention

The compounds of formula I provide several advantages over prior art materials
- by adding substituent chains and other groups they can be made more soluble, thus being suitable for spin coating or solution coating techniques, rather than vacuum deposition, to prepare thin films for use e.g. in electronic devices such as transistors,
- they can be made mesogenic or liquid crystalline, thus exhibiting a higher degree of order that leads to particularly high charge carrier mobility, in particular when being aligned in their meosphase into macroscopically uniform orientation,
- they combine the properties of a semiconducting material with those of a mesogenic material to give novel materials with a rigid, planar conjugated core and a flexible chain to increase solubility and to decrease the melting point, which show high charge carrier mobility when being aligned in their mesophase.

The compounds of formula I are useful as charge transport semiconductors in that they have high carrier mobilities. In particular, the introduction of alkyl side chains to the thienothiophene core improves the solubility and therefore the solution processibility of the compounds of formula I.

Particularly preferred are mesogenic or liquid crystalline compounds of formula I, wherein T is a mesogenic group. These compounds are particularly useful as semiconductors or charge transport materials, as they can be processed while in the highly ordered mesophase morphology, and readily aligned by conventional techniques in a preferred direction. Both smectic and nematic mesophase ordering allows close packing of molecular pi-electron systems, which maximises intermolecular charge transfer which occurs through a hopping mechanism between adjacent molecules. In this way charge trap sites at grain boundaries between regions of different orientation are reduced.

Thus, another object of the invention is a liquid crystal mixture comprising one or more compounds of formula I and optionally comprising one or more further compounds, wherein at least one of the compounds of formula I and the further compounds is mesogenic or liquid crystalline.

It is also possible to mix compounds of formula I with other mesogenic or liquid crystal monomers that are known from prior art, in order to induce or enhance liquid crystal phase behaviour.

It is also possible to mix compounds of formula I with liquid crystal polymers or with polymerisable compounds which have one or more polymerisable groups and which may also may also be mesogenic or liquid crystalline. For example, if the compounds of formula I are mixed with one or more polymerisable mesogenic or liquid crystal compounds, the oriented as described above can be permanently "frozen-in" by polymerising the mesogens, which can also create a structure with long range order, or "monodomain". Formation of a monodomain also maximises charge transfer by eliminating charge trap sites at grain boundaries, while the polymerisation also improves the mechanical properies of the film. Further, by crosslinking the mesogens, a highly stable structure results, which has an additional advantage of being impervious to subsequent processing solvents during device fabrication, thus allowing a wider range of solvents to be used in deposition of the next layer of the device by solution techniques. In addition, it is often observed that this crosslinking further densifies the film, leading to smaller intermolecular distances and improved charge transport.

Thus, another object of the invention is a polymerisable liquid crystal mixture comprising one or more compounds of formula I and comprising one or more further polymerisable compounds, wherein the further polymerisable compounds may also be mesogenic or liquid crystalline.

Polymerisable mesogenic compounds that are suitable as comonomers are known in prior art and disclosed for example in WO 93/22397; EP 0,261,712; DE 195,04,224; WO 95/22586 and WO 97/00600.

Another object of the present invention is an anisotropic polymer film with charge transport properties obtainable from a polymerisable liquid crystal mixture as defined above that is aligned in its liquid crystal phase into macroscopically ordered orientation and polymerised or crosslinked to fix the oriented state.

Particularly preferred are liquid crystal compounds of formula I, or liquid crystal mixtures comprising one or more compounds of formula I, that exhibit a nematic and/or smectic liquid crystal phase. For FET applications smectic materials are especially preferred. For OLED applications nematic or smectic materials are especially preferred.

Particularly preferred compounds of formula I are those wherein Z¹, A¹, Z², T, Z³, A² and Z⁴ form a conjugated system. Therein A¹ and A² are preferably arylene or heteroarylene and Z¹, Z², Z³ and Z⁴ are preferably a single bond or a conjugated link like for example -C≡C- or -CX¹=CX²-.

Further preferred compounds of formula I are those wherein
- m and o are 1 or 2,
- n is 1 and Z² is a single bond, -CX¹=CX²- or -C≡C-.
   Particularly preferred compounds of formula I are those of the following formulae

   R¹-Z¹-A¹-Z²-T-Z³-A²-Z⁴-R² I4

   R¹-Z¹-A¹-Z²-A¹-Z²-T-Z³-A²-Z⁴-R² I8

   R¹-Z¹-A¹-Z²-A¹-Z²-A¹-Z²-T-Z³-A¹-Z⁴-R² I17

   R¹-Z¹-A¹-Z²-A¹-Z²-T-Z³-A²-Z⁴-A²-Z⁴-R² I18

   R¹-Z¹-A¹-Z²-A¹-Z²-T-Z³-T-Z³-A²-Z⁴-R² I22

   R¹-Z¹-A¹-Z²-T-Z²-A¹-Z²-T-Z³-A²-Z⁴-R² I23

   R¹-Z¹-A¹-Z²-T-Z³-T-Z³-T-Z³-A²-Z⁴-R² I28

   wherein R¹, R², Z¹, Z², Z³, Z⁴, A¹, A² and T have, in case of multiple occurrence independently of each other, one of the meanings of formula I.
- CX¹=CX²- is preferably -CH=CH-, -CH=CF-, -CF=CH-, -CF=CF-, -CH=C(CN)- or -C(CN)=CH-.

R¹ and R² in formula I are preferably alkyl or alkoxy with 1 to 25 C atoms that is optionally fluorinated.

In the formulae shown above, R¹ to R⁸ are preferably selected from C₁-C₂₀-alkyl, C₁-C₂₀-fluoroalkyl, C₁-C₂₀-alkenyl, C₁-C₂₀-alkynyl, C₁-C₂₀-alkoxy, C₁-C₂₀-thioether, C₁-C₂₀-silyl, C₁-C₂₀-ester and C₁-C₂₀-amino.

If one of R or R¹ to R⁸ is an alkyl or alkoxy radical, i.e. where the terminal CH₂ group is replaced by -O-, this may be straight-chain or branched. It is preferably straight-chain, has 2, 3, 4, 5, 6, 7 or 8 carbon atoms and accordingly is preferably ethyl, propyl, butyl, pentyl, hexyl, heptyl, octyl, ethoxy, propoxy, butoxy, pentoxy, hexoxy, heptoxy, or octoxy, furthermore methyl, nonyl, decyl, undecyl, dodecyl, tridecyl, tetradecyl, pentadecyl, nonoxy, decoxy, undecoxy, dodecoxy, tridecoxy or tetradecoxy, for example.

Oxaalkyl, i.e. where one CH₂ group is replaced by -O-, is preferably straight-chain 2-oxapropyl (=methoxymethyl), 2- (=ethoxymethyl) or 3-oxabutyl (=2-methoxyethyl), 2-, 3-, or 4-oxapentyl, 2-, 3-, 4-, or 5-oxahexyl, 2-, 3-, 4-, 5-, or 6-oxaheptyl, 2-, 3-, 4-, 5-, 6- or 7-oxaoctyl, 2-, 3-, 4-, 5-, 6-, 7- or 8-oxanonyl or 2-, 3-, 4-, 5-, 6-,7-, 8- or 9-oxadecyl, for example.

Halogen is preferably F or Cl.

Particularly preferred are the following compounds 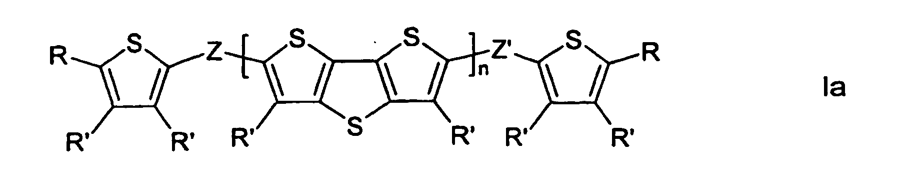 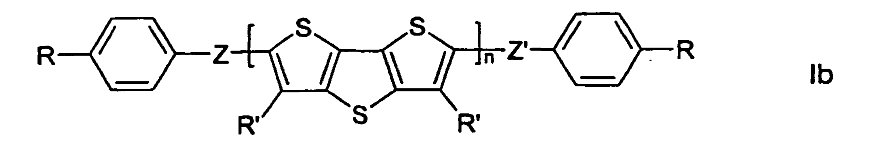 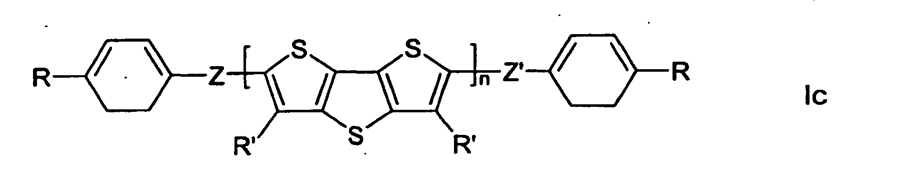 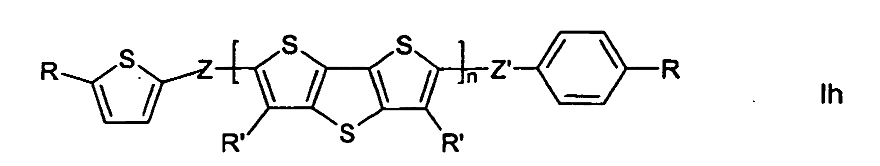 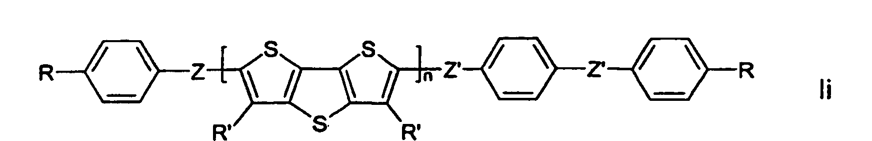 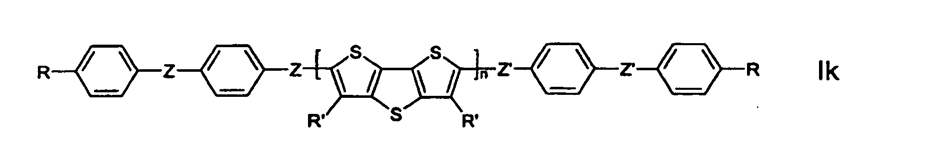 wherein n has the meaning of formula I,
- Z and Z': have independently of each other one of the meanings of Z' in formula II, and are preferably -CH=CH-, -CH=CF-, -CF=CH-, CH=CCI-, -CCl=CH-, -CF=CF-, -CCI=CCI-, -C≡C- or a single bond,
- Z": has one of the meanings of Z' in formula II, and is preferably -CH=CH-, -CH=CF-, -CF=CH-, CH=CCI-, -CCl=CH-, -CF=CF-, -CCI=CCI- or -C≡C-,
- R and R': have independently of each other one of the meanings of R¹ given above, and are preferably halogen or an optionally fluorinated alkyl group with 1 to 15 C atoms.

In case one of the groups A¹⁻², Z¹⁻⁴, R¹⁻⁸, R, R', R⁰, R⁰⁰, Z', Z" and T¹ appears more than once in a formula as shown above and below, the multiple groups may be identical or different, unless explicitly stated otherwise.

The compounds of formula I can be synthesized according to or in analogy to methods that are known to the skilled in the art and are described for example in F. de Jong and M. J. Janssen, J. Org. Chem., 1971, 36, 12, 1645; S. Inaoka and D. M. Collard, J. Mater. Chem., 1999, 9, 1719 or WO 99/12989. Furthermore, they can be prepared according to or in analogy to the following reaction schemes. DTT and BTT can be prepared according to scheme 1. 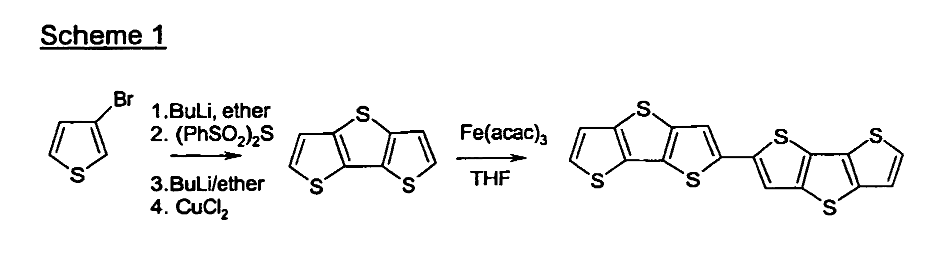 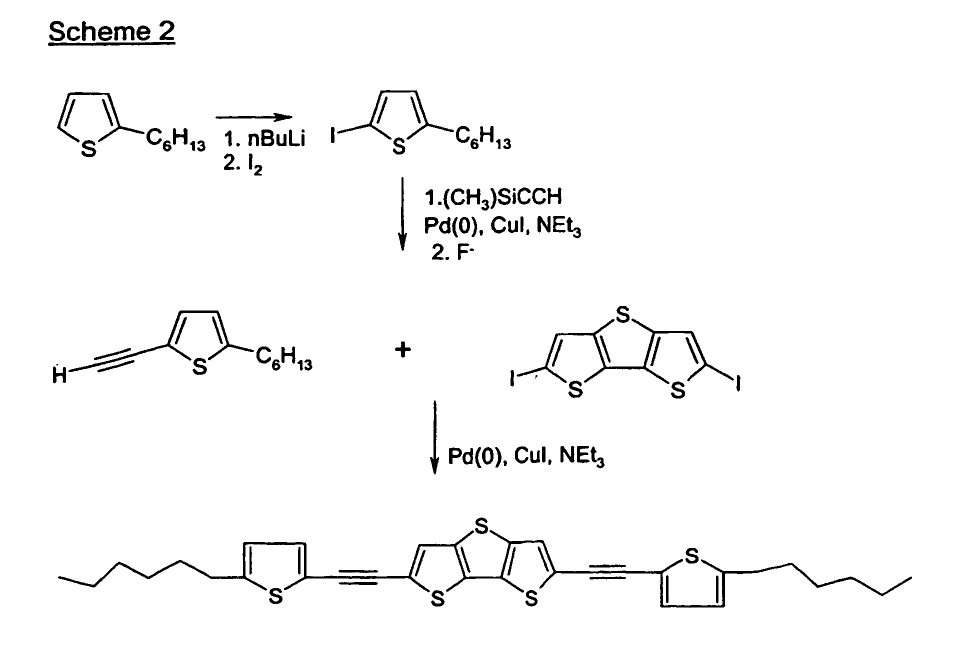 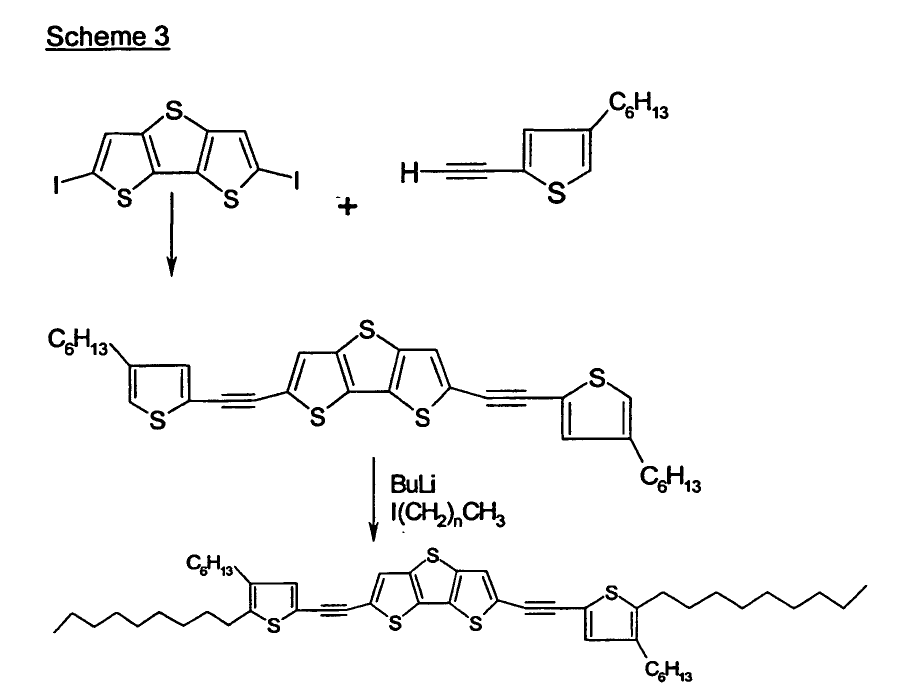 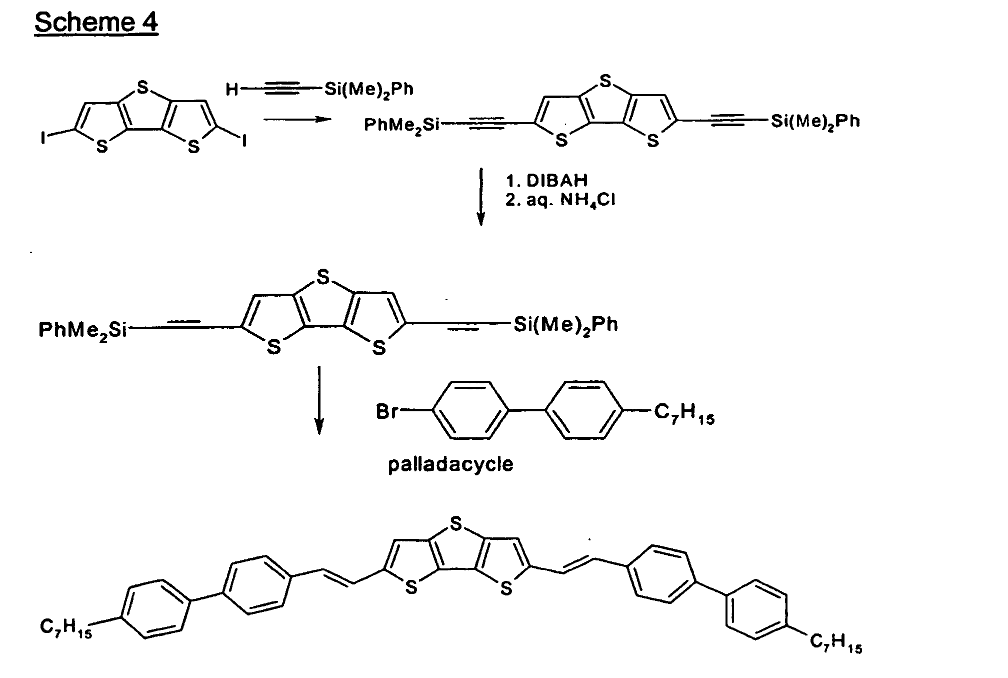 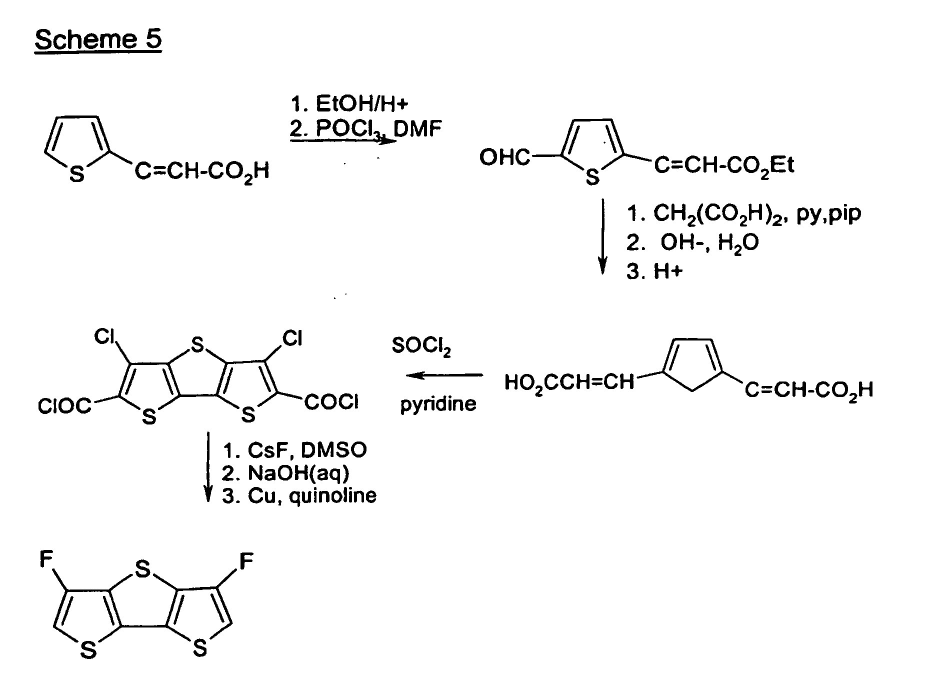 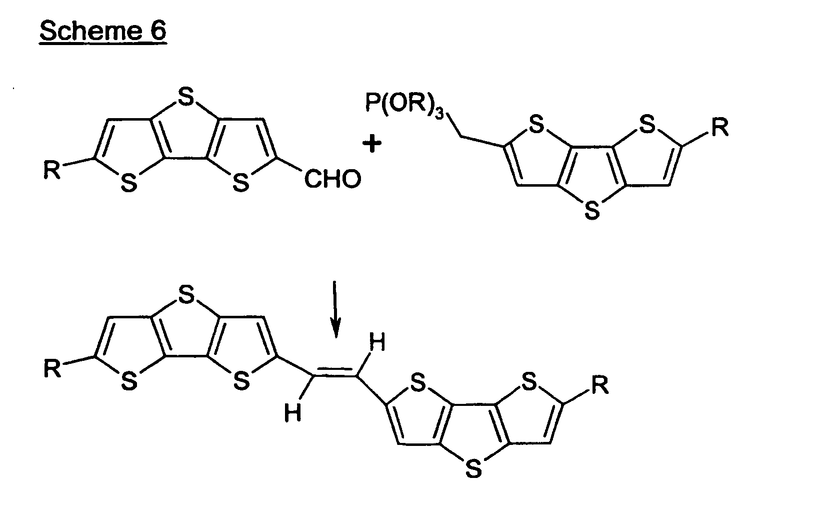 wherein R is C₁₋₂₀H₍₁₋₂₀₎₂₊₁

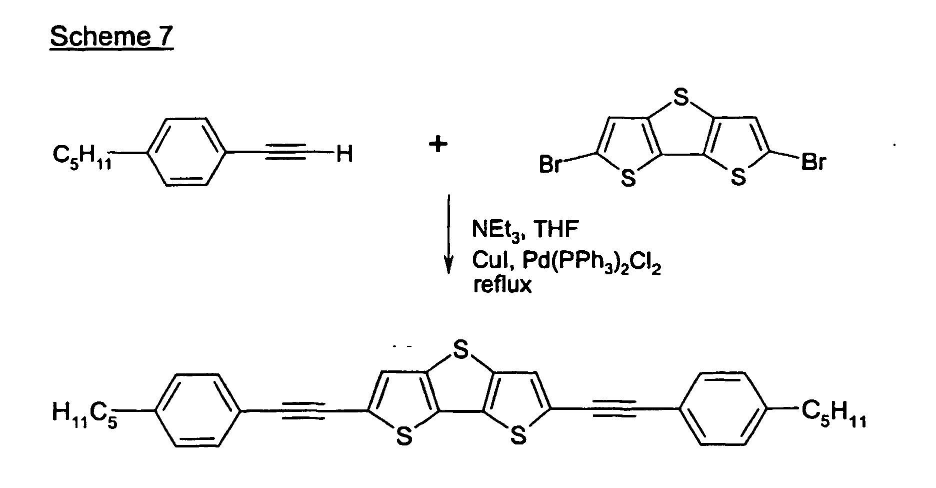

A further aspect of the invention relates to both the oxidised and reduced form of the compounds and materials according to this invention. Either loss or gain of electrons results in formation of a highly delocalised ionic form, which is of high conductivity. This can occur on exposure to common dopants. Suitable dopants and methods of doping are known to those skilled in the art, e.g. from EP 0 528 662, US 5,198,153 or WO 96/21659.

The doping process typically implies treatment of the semiconductor material with an oxidating or reducing agent in a redox reaction to form delocalised ionic centres in the material, with the corresponding counterions derived from the applied dopants. Suitable doping methods comprise for example exposure to a doping vapor in the atmospheric pressure or at a reduced pressure, electrochemical doping in a solution containing a dopant, bringing a dopant into contact with the semiconductor material to be thermally diffused, and ion-implantantion of the dopant into the semiconductor material.

When electrons are used as carriers, suitable dopants are for example halogens (e.g. I₂, Cl₂, Br₂, ICI, ICl₃, IBr and IF), Lewis acids (e.g. PF₅, AsF₅, SbF₅, BF₃, BCl₃, SbCl₅, BBr₃ and SO₃), protonic acids, organic acids, or amino acids (e.g. HF, HCI, HNO₃, H₂SO₄, HClO₄, FSO₃H and CISO₃H), transition metal compounds (e.g. FeCl₃, FeOCI, Fe(ClO₄)₃, Fe(4-CH₃C₆H₄SO₃)₃, TiCl₄, ZrCl₄, HfCl₄, NbF₅, NbCl₅, TaCl₅, MoF₅, MoCl₅, WF₅, WCl₆, UF₆ and LnCl₃ (wherein Ln is a lanthanoid), anions (e.g. Cl⁻, Br⁻, I⁻, I₃⁻, HSO₄⁻, SO₄²⁻, NO₃⁻, ClO₄⁻, BF₄⁻, PF₆⁻, AsF₆⁻, SbF₆⁻, FeCl₄⁻, Fe(CN)₆³⁻, and anions of various sulfonic acids, such as aryl-SO₃⁻). When holes are used as carriers, examples of dopants are cations (e.g. H⁺, Li⁺, Na⁺, K⁺, Rb⁺ and Cs⁺), alkali metals (e.g., Li, Na, K, Rb, and Cs), alkaline-earth metals (e.g., Ca, Sr, and Br), O₂, XeOF₄, (NO₂⁺) (SbF₆⁻), (NO₂⁺) (SbCl₆⁻), (NO₂⁺) (BF₄⁻), AgClO₄, H₂IrCl₆, La(NO₃)₃ · 6H₂O, FSO₂OOSO₂F, Eu, acetylcholine, R₄N⁺, (R is an alkyl group), R₄P⁺ (R is an alkyl group), R₆As⁺ (R is an alkyl group), and R₃S⁺ (R is an alkyl group).

The conducting form of the compounds and materials of the present invention can be used as an organic "metal" in applications, for example, but not limited to, charge injection layers and ITO planarising layers in organic light emitting diode applications, films for flat panel displays and touch screens, antistatic films, printed conductive substrates, patterns ot tracts in electronic applications such as printed circuit boards and condensers.

The mesogenic or liquid crystal compounds of formula I and the liquid crystal mixtures comprising compounds of formula I can be aligned in their liquid crystal state into homeotropic orientation, where the conjugated pi-electron systems are orthogonal to the direction of charge transport. This ensures that the intermolecular distances are minimised and hence then energy required to transport charge between molecules is minimised.

Alignment of the liquid crystal material can be achieved for example by treatment of the substrate onto which the material is coated, by shearing the material during or after coating, by application of a magnetic or electric field to the coated material, or by the addition of surface-active compounds to the liquid crystal material. Reviews of alignment techniques are given for example by I. Sage in "Thermotropic Liquid Crystals", edited by G. W. Gray, John Wiley & Sons, 1987, pages 75-77, and by T. Uchida and H. Seki in "Liquid Crystals - Applications and Uses Vol. 3", edited by B. Bahadur, World Scientific Publishing, Singapore 1992, pages 1-63. A review of alignment materials and techniques is given by J. Cognard, Mol. Cryst. Liq. Cryst. 78, Supplement 1 (1981), pages 1-77.

In case of polymerisable materials, polymerisation can be achieved by exposure to heat or actinic radiation. Actinic radiation means irradiation with light, like UV light, IR light or visible light, irradiation with X-rays or gamma rays or irradiation with high energy particles, such as ions or electrons. Preferably polymerisation is carried out by UV irradiation at a non-absorbing wavelength. As a source for actinic radiation for example a single UV lamp or a set of UV lamps can be used. When using a high lamp power the curing time can be reduced. Another possible source for actinic radiation is a laser, like e.g. a UV laser, an IR laser or a visible laser.

Polymerisation is preferably carried out in the presence of an initiator absorbing at the wavelength of the actinic radiation. For example, when polymerising by means of UV light, a photoinitiator can be used that decomposes under UV irradiation to produce free radicals or ions that start the polymerisation reaction. When curing polymerisable materials with acrylate or methacrylate groups, preferably a radical photoinitiator is used, when curing polymerisable materials with vinyl, epoxide and oxetane groups, preferably a cationic photoinitiator is used. It is also possible to use a polymerisation initiator that decomposes when heated to produce free radicals or ions that start the polymerisation. As a photoinitiator for radical polymerisation for example the commercially available Irgacure 651, Irgacure 184, Darocure 1173 or Darocure 4205 (all from Ciba Geigy AG) can be used, whereas in case of cationic photopolymerisation the commercially available UVI 6974 (Union Carbide) can be used.

The polymerisable material can additionally comprise one or more other suitable components such as, for example, catalysts, sensitizers, stabilizers, inhibitors, chain-transfer agents, co-reacting monomers, surface-active compounds, lubricating agents, wetting agents, dispersing agents, hydrophobing agents, adhesive agents, flow improvers, defoaming agents, deaerators, diluents, reactive diluents, auxiliaries, colourants, dyes or pigments.

The compounds of formula I and the mixtures obtained thereof are useful as optical, electronic and semiconductor materials, in particular as charge transport materials in field effect transistors (FETs) e.g. as components of integrated circuitry, ID tags or TFT applications. Alternatively, they may be used in organic light emitting diodes (OLEDs) in electroluminescent display applications or as backlight of e.g. liquid crystal displays, as photovoltaics or sensor materials, and for other semiconductor applications, as electrode materials in batteries, as photoconductors and for electrophotographic applications like electrophotographic recording.

FETs comprising compounds of formula I or mixtures or polymers comprising them are suitable for example as ID tags, containing specific information in clothing, food containers and other consumer products. In security applications they are suitable for use in field effect transistors for ID tags or security markings to authenticate and prevent counterfeiting of documents of value like banknotes, credit cards or ID cards, national ID documents, licenses or any product with money value, like stamps, tickets, shares, cheques etc..

The materials of the present invention are useful as optical, electronic and semiconductor materials, in particular as charge transport materials in field effect transistors (FETs), as photovoltaics or sensor materials, for electrophotographic recording, and for other semiconductor applications. Such FETs, where an organic semiconductive material is arranged as a film between a gate-dielectric and a drain and a source electrode, are generally known e.g. from US 5,892,244, WO 00/79617, US 5,998,804, and from the references cited in the background and prior art chapter. Due to the advantages, like low cost production using the solubility properties of the compounds according to the invention and thus the processibility of large surfaces, preferred applications of these FETs are such as integrated circuitry, TFT-displays and security applications.

Alternatively, the materials according to the invention may be used in organic light emitting devices or diodes (OLEDs), e.g. in display applications or as backlight of e.g. liquid crystal displays. Common OLEDs are realized using multilayer structures. An emission layer is generally sandwiched between one or more electron-transport and/or hole-transport layers. By applying an electric voltage electrons and holes as charge carriers move towards the emission layer where their recombination leads to the excitation and hence luminescence of the lumophor units contained in the emission layer. The inventive compounds, materials and films may be employed in one or more of the charge transport layers and/ or in the emission layer, corresponding to their electrical and/ or optical properties. Furthermore their use within the emission layer is especially advantageous, if the compounds, materials and films according to the invention show electroluminescent properties themselves or comprise electroluminescent groups or compounds. The selection, characterization as well as the processing of suitable monomeric, oligomeric and polymeric compounds or materials for the use in OLEDs is generally known by a person skilled in the art, see e. g. Meerholz, Synthetic Materials, 111-112, 2000, 31-34, Alcala, J. Appl. Phys., 88, 2000, 7124-7128 and the literature cited therein.

According to another use, the inventive compounds, materials or films, especially those which show photoluminescent properties, may be employed as materials of light sources, e.g. of display devices such as described in EP 0 889 350 A1 or by C. Weder et al., Science, 279, 1998, 835-837.

Furthermore, the compounds of the present invention are useful as high birefringence compounds added to liquid crystalline compositions in order to increase birefringence. For this purpose, they do not need to have a mesophase themselves, but a similar shape to conventional liquid crystals in order to dissolve and not to detract from the liquid crystal properties of the composition.

In the foregoing and in the following examples, unless otherwise indicated, all temperatures are set forth uncorrected in degrees Celsius and all parts and percentages are by weight. The following abbreviations are used to illustrate the liquid crystalline phase behaviour of the compounds: K = crystalline; N = nematic; S = smectic; Ch = cholesteric; I = isotropic. The numbers between the symbols indicate the phase transition temperatures in °C.

### Example 1

2,6-Bis-(4-pentyl-phenylethynyl)-dithieno[3,2-*b*:2',3'-*d*]thiophene (1) was prepared according to scheme 7 and as described below

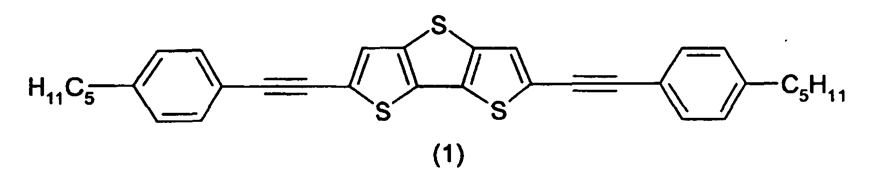

2,6-Dibromo-dithieno[3,2-*b*;2',3'-*d*]thiophene was prepared according to a procedure described in G. F. Pedulli, M. Tiecco, M. Guera, G. Martelli and P. Zanirato, *J. C. S. Perkin II*, 1978, 212. 2,6-Dibromo-dithieno[3,2-*b*;2',3'-*d*]thiophene (0.5 g, 1.4 mmol), triethylamine (15 ml), and a catalytic amount of palladium bis(triphenylphosphine) dichloride and copper iodide were stirred under nitrogen in tetrahydrofuran. The solution was warmed to 60°C and 4-pentylphenylacetylene (1.1 g, 6.4 mmol) dissolved in tetrahydrofuran (30 ml) was added dropwise over a period of 2 hours. The solution was heated under reflux overnight. The brown solution was poured in to dichloromethane, washed with water, the chlorinated phase was removed, dried over sodium sulphate and evaporated to dryness. The residue was purified by flash column chromatography using petroleum (80-100) followed by dichloromethane as eluant.

Evaporation of the appropriate fractions yielded (1) as a bright yellow solid (320 mg). ¹H NMR and ¹³C NMR showed expected signals.

The following transitions and phases were observed by optical microscopy:
K-134-N-235-I (first heat)
I-234-N-110-K (first cool)
K-134-N-234-I (second heat)

### Example 2

Compound (2) was prepared as follows:

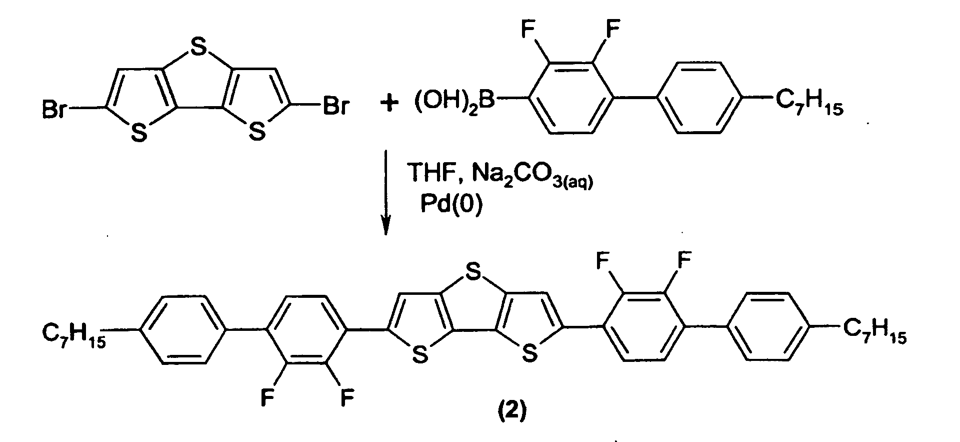

2,6-Dibromo-dithieno[3,2-*b*;2',3'-*d*]thiophene (1.0 g, 2.8 mmol) and 4'-heptylphenyl-2,3-difluorophenyl benzoic acid (2.1 g), sodium carbonate (1.07 g, 10.1 mmol in 20 ml water) and a catalytic amount of palladium bis(triphenylphosphine) dichloride were stirred under reflux in tetrahydrofuran. After 16 h, the brown solution was poured in to dichloromethane, washed with water, the chlorinated phase was removed, dried over sodium sulphate and evaporated to dryness. The residue was purified by flash column chromatography using petroleum (80-100) followed by dichloromethane as eluant. Evaporation of the appropriate fractions yielded (2) as a bright yellow solid (430 mg). ¹H and ¹³C NMR showed expected signals.

The following transitions and phases were observed by optical microscopy:
K-123-N-262-I (first heat)
I-256-N-112-K (first cool)

## Claims

1. Thienothiophenes of formula I
R¹-Z¹-(A¹-Z²)ₘ-(T-Z³)ₙ-(A²-Z⁴)ₒ-R² I
wherein
R¹ and R² are independently of each other straight chain or branched alkyl with 1 to 20 C-atoms, which may be unsubstituted, mono- or polysubstituted by F, Cl, Br, I or CN, it being also possible for one or more non-adjacent CH₂ groups to be replaced, in each case independently from one another, by -O-, -S-, -NH-, -NR⁰-, -SiR⁰R⁰⁰-, -CO-, -COO-, -OCO-, -OCO- O-, -S-CO-, -CO-S-, -CH=CH- or -C≡C- in such a manner that O and/or S atoms are not linked directly to one another,
R⁰ and R⁰⁰ are independently of each other H or alkyl with 1 to 12 C-atoms,
A¹ and A² are independently of each other 1,4-phenylene or thiophene-2,5-diyl, it being possible for these groups to be unsubstituted, mono- or polysubstituted by L, with L being halogen, CN, SCN, NO₂, SF₅ or an alkyl, alkoxy, alkylcarbonyl or alkoxycarbonyl group with 1 to 12 C atoms, wherein one or more H atoms may be substituted with F or Cl,
Z¹, Z², Z³ and Z⁴ are independently of each other -CH=CR⁰-, -CX¹=CX²-, -C≡C- or a single bond,
X¹ and X² are independently of each other H, F, Cl or CN,
T is a group of formula IIa 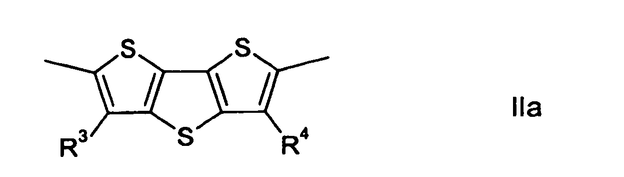
R³ and R⁴ are H or have one of the meanings of R¹,
m and o are independently of each other 1, 2 or 3, and
n is 1.

2. Thienothiophenes according to claim 1, **characterized in that** they are liquid crystalline.

3. Thienothiophenes according to claim 1 or 2, selected from the following subformulae
R¹-Z¹-A¹-Z²-T-Z³-A²-Z⁴-R² I4
R¹-Z¹-A¹-Z²-A¹-Z²-T-Z³-A²-Z⁴-R² I8
R¹-Z¹-A¹-Z²-A¹-Z²-A¹-Z²-T-Z³-A¹-Z⁴-R² I17
R¹-Z¹-A¹-Z²-A¹-Z²-T-Z³-A²-Z⁴-A²-Z⁴-R² I18
wherein R¹, R², Z¹, Z², Z³, Z⁴, A¹, A² and T have, in case of multiple occurrence independently of each other, one of the meanings of formula I.

4. Thienothiophenes according to at least one of claims 1 to 3, **characterized in that** R¹ and R² are alkyl or alkoxy with 1 to 25 C atoms that is optionally fluorinated.

5. Thienothiophenes according to at least one of claims 1 to 4, **characterized in that** A¹ and A² are 1,4-phenylene that is unsubstituted, mono- or polysubstituted by L.

6. Thienothiophenes according to at least one of claims 1 to 5, **characterized in that** Z¹, Z², Z³ and Z⁴ are -C≡C- or a single bond.

7. Thienothiophenes according to at least one of claims 1 to 6, **characterized in that** they are selected from the following formulae 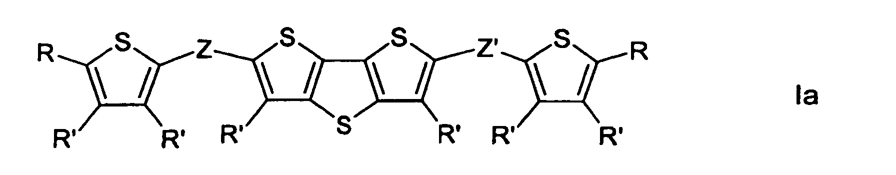 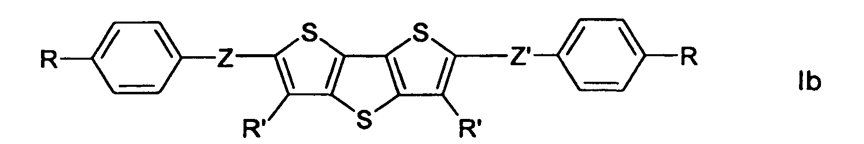 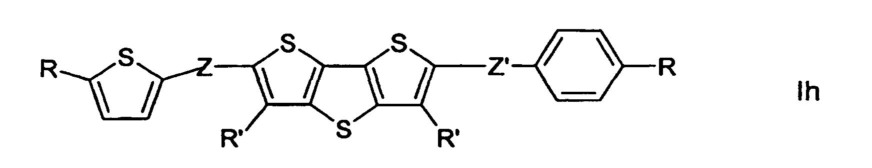 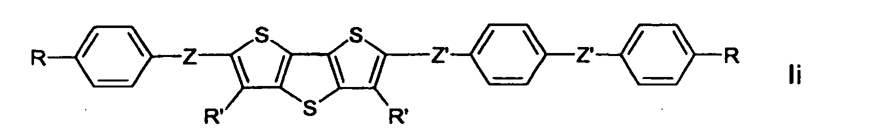 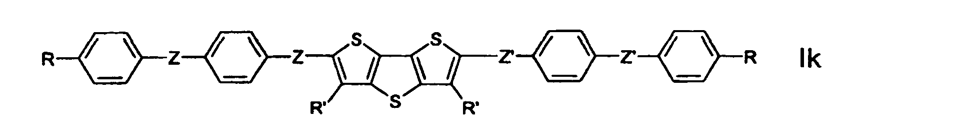 wherein
Z and Z' are independently of each other -CH=CH-, -C≡C- or a single bond,
R have independently of each other one of the meanings of R¹ of claims 1 to 5.
R' have independently of each other one of the meanings of R³ of claims 1 to 5.

8. Liquid crystal mixture comprising one or more thienothiophenes according to at least one of claims 1 to 7.

9. Liquid crystal mixture or thienothiophene according to at least one of claims 1 to 8, **characterized in that** it exhibits a nematic or smectic liquid crystal phase.

10. Polymerisable liquid crystal mixture comprising at least one mixture or thienothiophene according to at least one of claims 1 to 9, **characterized in that** further comprises one or more liquid crystal polymers or one or more polymerisable compounds which can also be mesogenic or liquid crystalline.

11. Anisotropic polymer film with charge transport properties, **characterized in that** it is obtainable from a mixture according to claim 10 that is aligned in its liquid crystal phase into macroscopically ordered orientation and polymerised or crosslinked to fix the oriented state.

12. Liquid crystal mixture, thienothiophene or polymer film according to at least one of claims 1 to 11, wherein the mesogenic or liquid crystal compounds are aligned in their liquid crystal state into homeotropic orientation.

13. Use of mixtures, polymer films or thienothiophenes of claims 1 to 12 as semiconductors or charge transport materials in optical, electrooptical or electronic devices, like for example components of integrated circuitry, field effect transistors (FET) for example as thin film transistors in flat panel display applications or for Radio Frequency Identification (RFID) tags, and in semiconducting components for organic light emitting diode (OLED) applications, electroluminescent display devices, backlights, photovoltaic or sensor devices, as electrode materials in batteries, as photoconductors and for electrophotographic applications.

14. Field effect transistor, OLED, electroluminescent device, RFID tag, backlight, photovoltaic or sensor device, photoconductor or electrophotographic recording device comprising a mixture, polymer film or thienothiophene of claims 1 to 12.

15. Security marking or device comprising a mixture, polymer film or thienothiophene of claims 1 to 12 or a FET or RFID according to claim 14.

16. Liquid crystal mixture, polymer film or thienothiophene according to at least one of claims 1 to 12, wherein the thienothiophenes are oxidatively or reductively doped to form conducting ionic species.

17. Charge injection layer, planarising layer, antistatic film or conducting substrate or pattern for electronic applications or flat panel displays, comprising a mixture, polymer film or thienothiophene according to claim 16.

## Patentansprüche

1. Thienothiophene der Formel I
R¹-Z¹-(A¹-Z²)ₘ-(T-Z³)ₙ-(A²-Z⁴)ₒ-R² I
worin
R¹ und R² unabhängig voneinander geradkettiges oder verzweigtes Alkyl mit 1 bis 20 C-Atomen bedeuten, das unsubstituiert oder ein- oder mehrfach durch F, Cl, Br, I oder CN substituiert sein kann, wobei gegebenenfalls eine oder mehrere nicht benachbarte CH₂-Gruppen jeweils unabhängig voneinander durch -O-, -S-, -NH-, -NR⁰-, -SiR⁰R⁰⁰-, -CO-, -COO-, -OCO-, -OCO-O-, -S-CO-, -CO-S-, -CH=CH- oder -C=C- so ersetzt sind, dass O- und/oder S-Atome nicht direkt miteinander verknüpft sind,
R⁰ und R⁰⁰ unabhängig voneinander H oder Alkyl mit 1 bis 12 C-Atomen bedeuten,
A¹ und A² unabhängig voneinander 1,4-Phenylen oder Thiophen-2,5-diyl bedeuten, wobei diese Gruppen unsubstituiert oder ein- oder mehrfach durch L substituiert sein können und L Halogen, CN, SCN, NO₂, SF₅ oder eine Alkyl-, Alkoxy-, Alkylcarbonyl- oder Alkoxycarbonylgruppe mit 1 bis 12 C-Atomen bedeutet, worin ein oder mehrere H-Atome gegebenenfalls mit F oder Cl substituiert sind,
Z¹, Z², Z³ und Z⁴ unabhängig voneinander -CH=CR⁰-, -CX¹=CX²-, -C≡C- oder eine Einfachbindung bedeuten,
X¹ und X² unabhängig voneinander H, F, Cl oder CN bedeuten,
T eine Gruppe der Formel IIa 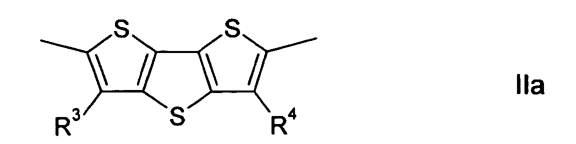 bedeutet,
R³ und R⁴ H bedeuten oder eine der Bedeutungen von R¹ besitzen,
m und o unabhängig voneinander 1, 2 oder 3 bedeuten und
n 1 ist.

2. Thienothiophene nach Anspruch 1, **dadurch gekennzeichnet, dass** sie flüssigkristallin sind.

3. Thienothiophene nach Anspruch 1 oder 2, ausgewählt aus den folgenden Teilformeln
R¹-Z¹-A¹-Z²-T-Z³-A²-Z⁴-R² I4
R¹-Z¹-A¹-Z²-A¹-Z²-T-Z³-A²-Z⁴-R² I8
R¹-Z¹-A¹-Z²-A¹-Z²-A¹-Z²-T-Z³-A¹-Z⁴-R² I17
R¹-Z¹-A¹-Z²-A¹-Z²-T-Z³-A²-Z⁴-A²-Z⁴-R² I18
worin R¹, R², Z¹, Z², Z³, Z⁴, A¹, A² und T, bei mehrfachem Auftreten unabhängig voneinander, eine der Bedeutungen der Formel I besitzen.

4. Thienothiophene nach mindestens einem der Ansprüche 1 bis 3, **dadurch gekennzeichnet, dass** R¹ und R² Alkyl oder Alkoxy mit 1 bis 25 C-Atomen bedeuten, das gegebenenfalls fluoriert ist.

5. Thienothiophene nach mindestens einem der Ansprüche 1 bis 4, **dadurch gekennzeichnet, dass** A¹ und A² 1,4-Phenylen bedeuten, das unsubstituiert oder ein- oder mehrfach durch L substituiert ist.

6. Thienothiophene nach mindestens einem der Ansprüche 1 bis 5, **dadurch gekennzeichnet, dass** Z¹, Z², Z³ und Z⁴ -C≡C- oder eine Einfachbindung bedeuten.

7. Thienothiophene nach mindestens einem der Ansprüche 1 bis 6, **dadurch gekennzeichnet, dass** sie ausgewählt sind aus den folgenden Formeln 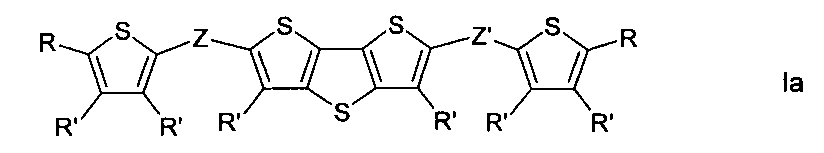 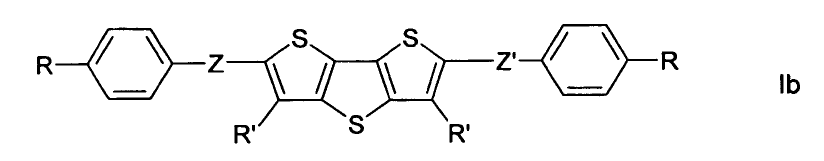 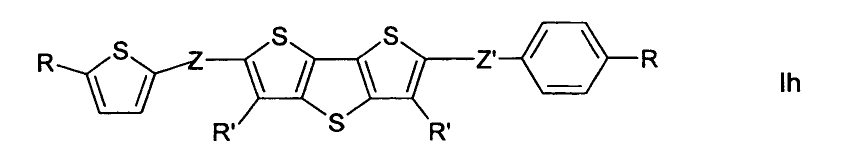 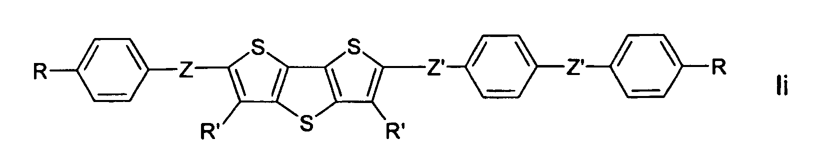 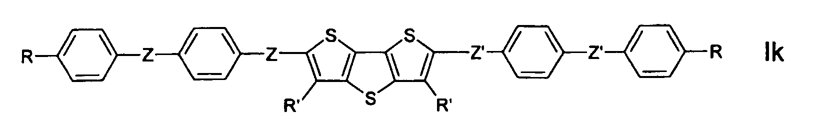 worin
Z und Z' unabhängig voneinander -CH=CH-, -C≡C- oder eine Einfachbindung bedeuten,
R unabhängig voneinander eine der Bedeutungen von R¹ der Ansprüche 1 bis 5 besitzen,
R' unabhängig voneinander eine der Bedeutungen von R³ der Ansprüche 1 bis 5 besitzen.

8. Flüssigkristallmischung enthaltend ein oder mehrere Thienothiophene nach mindestens einem der Ansprüche 1 bis 7.

9. Flüssigkristallmischung oder Thienothiophen nach mindestens einem der Ansprüche 1 bis 8, **dadurch gekennzeichnet, dass** sie bzw. es eine nematische oder smektische Flüssigkristallphase aufweist.

10. Polymerisierbare Flüssigkristallmischung enthaltend mindestens eine Mischung oder ein Thienothiophen nach mindestens einem der Ansprüche 1 bis 9, **dadurch gekennzeichnet, dass** sie weiterhin ein oder mehrere Flüssigkristallpolymere oder eine oder mehrere polymerisierbare Verbindungen enthält, die auch mesogen oder flüssigkristallin sein können.

11. Anisotrope Polymerfolie mit ladungstransportierenden Eigenschaften, **dadurch gekennzeichnet, dass** sie erhältlich ist aus einer Mischung nach Anspruch 10, die in ihrer Flüssigkristallphase in makroskopisch geordnete Ausrichtung orientiert und zur Fixierung des ausgerichteten Zustandes polymerisiert oder vernetzt ist.

12. Flüssigkristallmischung, Thienothiophen oder Polymerfolie nach mindestens einem der Ansprüche 1 bis 11, worin die mesogenen oder Flüssigkristallverbindungen in ihrem Flüssigkristallzustand in homeotrope Ausrichtung orientiert sind.

13. Verwendung von Mischungen, Polymerfolien oder Thienothiophenen der Ansprüche 1 bis 12 als Halbleiter oder Ladungstransportmaterialien in optischen, elektrooptischen oder elektronischen Vorrichtungen, wie beispielsweise Komponenten integrierter Schaltungen, Feldeffekttransistoren (FET) z.B. als Dünnfilmtransistoren in Flachbildschirm-Anwendungen oder für RFID-Tags (Radio Frequency Identification), und in Halbleiterkomponenten für Anwendungen mit organischen Leuchtdioden (OLED), Elektrolumineszenzanzeigevorrichtungen, Hintergrundbeleuchtungen, photovoltaischen oder Sensorvorrichtungen, als Elektrodenmaterialien in Batterien, als Photoleiter und für elektrophotographische Anwendungen.

14. Feldeffekttransistor, OLED, Elektrolumineszenzvorrichtung, RFID-Tag, Hintergrundbeleuchtung, photovoltaische oder Sensorvorrichtung, Photoleiter oder elektrophotographische Aufzeichnungsvorrichtung enthaltend eine Mischung, eine Polymerfolie oder ein Thienothiophen der Ansprüche 1 bis 12.

15. Sicherheitsmarkierung oder -vorrichtung enthaltend eine Mischung, eine Polymerfolie oder ein Thienothiophen der Ansprüche 1 bis 12, oder einen FET oder ein RFID-Tag nach Anspruch 14.

16. Flüssigkristallmischung, Polymerfolie oder Thienothiophen nach mindestens einem der Ansprüche 1 bis 12, worin die Thienothiophene oxidativ oder reduktiv dotiert sind, so dass sie leitende Ionenspezies bilden.

17. Ladungsinjektionsschicht, Planarisierungsschicht, Antistatikfolie oder leitendes Substrat oder Muster für Elektronikanwendungen oder Flachbildschirme, enthaltend eine Mischung, eine Polymerfolie oder ein Thienothiophen nach Anspruch 16.

## Revendications

1. Thiénothiophènes de la formule I
R¹-Z¹-(A¹-Z²)ₘ-(T-Z³)ₙ-(A²-Z⁴)ₒ-R² I
dans laquelle
R¹ et R² sont indépendamment l'un de l'autre alkyle en chaîne droite ou ramifié avec de 1 à 20 atomes de C, qui peut être non substitué, mono- ou polysubstitué par F, Cl, Br, I ou CN, étant entendu qu'il est également possible qu'un ou plusieurs groupes CH₂ non adjacents soient remplacés, dans chaque cas indépendamment les uns des autres, par -O-, -S-, -NH-, -NR⁰-, -SiR⁰R⁰⁰-, -CO-, -COO-, -OCO-, -OCO-O-, -S-CO-, -CO-S-, -CH=CH- ou -C≡C- de telle sorte que des atomes de O et/ou de S ne soient pas liés directement les uns aux autres,
R⁰ et R⁰⁰ sont indépendamment l'un de l'autre H ou alkyle avec de 1 à 12 atomes de C,
A¹ et A² sont indépendamment l'un de l'autre 1,4-phénylène ou thiophène-2,5-diyle, étant entendu qu'il est possible que ces groupes soient non substitués, mono- ou polysubstitués par L, L étant halogène, CN, SCN, NO₂, SF₅ ou un groupe alkyle, alkoxy, alkylcarbonyle ou alkoxycarbonyle avec de 1 à 12 atomes de C, où un ou plusieurs atomes de H peuvent être substitués par F ou CI,
Z¹, Z², Z³ et Z⁴ sont indépendamment les uns des autres -CH=CR⁰-, -CX¹=CX²-, -C=C- ou une liaison simple,
X¹ et X² sont indépendamment l'un de l'autre H, F, Cl ou CN,
T est un groupe de la formule IIa 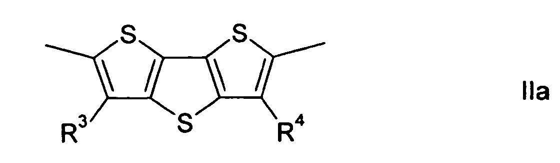
R³ et R⁴ sont H ou présentent l'une des significations de R¹,
m et o sont indépendamment l'un de l'autre 1, 2 ou 3, et
n est 1.

2. Thiénothiophènes selon la revendication 1, **caractérisés en ce qu'**ils sont cristallins liquides.

3. Thiénothiophènes selon la revendication 1 ou 2, choisis parmi les sous-formules qui suivent
R¹-Z¹-A¹-Z²-T-Z³-A²-Z⁴-R² I4
R¹-Z¹-A¹-Z²-A¹-Z²-T-Z³-A²-Z⁴-R² I8
R¹-Z¹-A¹-Z²-A¹-Z²-A¹-Z²-T-Z³-A¹-Z⁴-R² I17
R¹-Z¹-A¹-Z²-A¹-Z²-T-Z³-A²-Z⁴-A²-Z⁴-R² I18
dans lesquelles R¹, R², Z¹, Z², Z³, Z⁴, A¹, A² et T présentent, dans le cas de multiples occurrences indépendamment les unes des autres, l'une des significations de la formule I.

4. Thiénothiophènes selon au moins l'une des revendications 1 à 3, **caractérisés en ce que** R¹ et R² sont alkyle ou alkoxy avec de 1 à 25 atomes de C qui est en option fluoré.

5. Thiénothiophènes selon au moins l'une des revendications 1 à 4, **caractérisés en ce que** A¹ et A² sont 1,4-phénylène qui est non substitué, mono- ou polysubstitué par L.

6. Thiénothiophènes selon au moins l'une des revendications 1 à 5, **caractérisés en ce que** Z¹, Z², Z³ et Z⁴ sont -C≡C- ou une liaison simple.

7. Thiénothiophènes selon au moins l'une des revendications 1 à 6, **caractérisés en ce qu'**ils sont choisis parmi les formules qui suivent 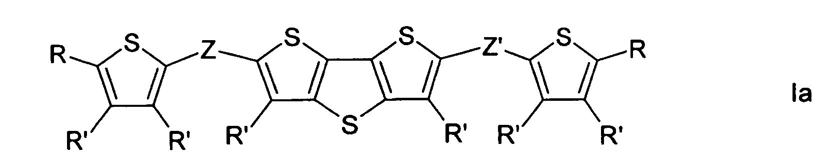 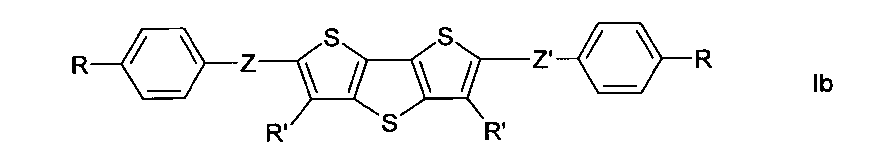 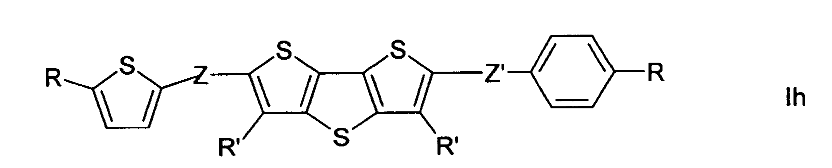 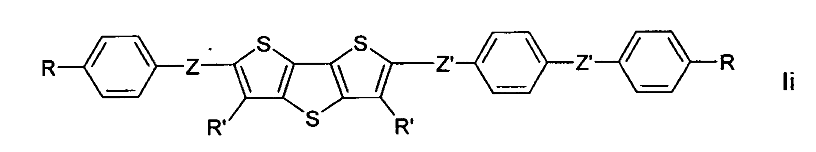 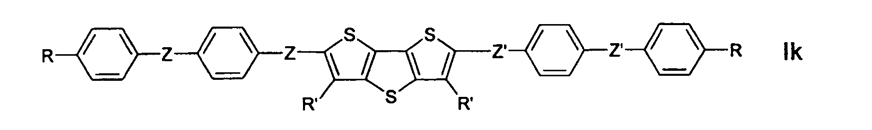 dans lesquelles
Z et Z' sont indépendamment l'un de l'autre -CH=CH-, -C≡C- ou une liaison simple,
R présentent indépendamment les uns des autres l'une des significations de R¹ des revendications 1 à 5,
R' présentent indépendamment les uns des autres l'une des significations de R³ des revendications 1 à 5.

8. Mélange de cristaux liquides comprenant un ou plusieurs thiénothiophènes selon au moins l'une des revendications 1 à 7.

9. Mélange de cristaux liquides ou thiénothiophène selon au moins l'une des revendications 1 à 8, **caractérisé en ce qu'**il présente une phase de cristal liquide nématique ou smectique.

10. Mélange de cristaux liquides polymérisable comprenant au moins un mélange ou thiénothiophène selon au moins l'une des revendications 1 à 9, **caractérisé en ce qu'**il comprend en outre un ou plusieurs polymères de cristal liquide ou un ou plusieurs composés polymérisables qui peuvent également être mésogènes ou cristallins liquides.

11. Film en polymère anisotrope présentant des propriétés de transport de charges, **caractérisé en ce qu'**il peut être obtenu à partir d'un mélange selon la revendication 10 qui est aligné dans sa phase de cristal liquide selon une orientation ordonnée macroscopiquement et qui est polymérisé ou réticulé afin de fixer l'état orienté.

12. Mélange de cristaux liquides, thiénothiophène ou film en polymère selon au moins l'une des revendications 1 à 11, dans lequel les composés mésogènes ou de cristal liquide sont alignés dans leur état de cristal liquide selon une orientation homéotrope.

13. Utilisation de mélanges, de films en polymère ou de thiénothiophènes des revendications 1 à 12 en tant que semiconducteurs ou matériaux de transport de charges dans des dispositifs optiques, électrooptiques ou électroniques, tels que par exemple des composants de circuit intégré, des transistors à effet de champ (FET) par exemple en tant que transistors à film mince dans des applications d'affichage à écran plat ou pour des étiquettes d'identification radiofréquence (IDRF), et dans des composants de semiconduction pour des applications par diodes émettrices de lumières organiques (DELO), des dispositifs d'affichage électroluminescents, des éclairages arrière, des dispositifs photovoltaïques ou de capteur, en tant que matériaux d'électrode dans des accumulateurs, en tant que photoconducteurs et pour des applications électrophotographiques.

14. Transistor à effet de champ, DELO, dispositif électroluminescent, étiquette IDRF, éclairage arrière, dispositif photovoltaïque ou de capteur, dispositif d'enregistrement photoconducteur ou électrophotographique comprenant un mélange, un film en polymère ou un thiénothiophène des revendications 1 à 12.

15. Marquage ou dispositif de sécurité comprenant un mélange, un film en polymère ou un thiénothiophène des revendications 1 à 12 ou un FET ou une IDRF selon la revendication 14.

16. Mélange de cristaux liquides, film en polymère ou thiénothiophène selon au moins l'une des revendications 1 à 12, dans lequel les thiénothiophènes sont dopés de façon oxydante ou réductrice pour former des espèces ioniques de conduction.

17. Couche d'injection de charges, couche de planarisation, film antistatique ou substrat ou motif de conduction pour des applications électroniques ou des affichages à écran plat, comprenant un mélange, un film en polymère ou un thiénothiophène selon la revendication 16.
